# EUROPEAN PATENT APPLICATION

(11) **EP 4 545 073 A1**
(43) Date of publication of application: **30.04.2025**
(21) Application number: 23842473.3
(22) Date of filing: 18.09.2023
(51) Int. Cl.: A61K 9/22, A61K 9/20, A61K 9/28

(54) **BIPHASIC CONTROLLED-RELEASE FORMULATION AND PREPARATION METHOD THEREFOR**

(30) Priority: 21.07.2022 CN 202210874158; 21.07.2023 CN 202310905085
(71) Applicant: Overseas Pharmaceuticals, Ltd., China 510530 (CN)
(72) Inventor: HUANG, Xiaofeng, Guangzhou, Guangdong 510530 (CN); ZHANG, Chenliang, Guangzhou, Guangdong 510530 (CN); WEN, Xiaoguang, Guangzhou, Guangdong 510530 (CN)
(74) Representative: Murgitroyd & Company
(86) International application number: PCT/CN2023/119277
(87) International publication number: WO 2024/017411

(57) **Abstract**

The present invention relates to a biphasic controlled-release preparation and preparation thereof. The biphasic controlled-release preparation can take effect quickly *in vivo,* quickly reach a peak value of blood drug concentration, then keep stable release, maintain stable blood drug concentration and reduce the fluctuation between wave peaks and wave valleys. The controlled-release preparation consists of three parts, namely an immediate-release layer, a tablet core layer and a blocking layer. The immediate-release layer is rapidly dissolved *in vitro,* and the combination of the remaining tablet core layer and the blocking layer can maintain the zero-order release *in vitro.*

## Description

### TECHNICAL FIELD

The present invention relates to pharmaceutical technology, and in particular to a biphasic controlled-release preparation and a preparation method thereof.

### BACKGROUND

In recent years, with the deepening of theoretical research on drug delivery systems and the development of polymer materials science, the research on drug delivery systems is increasing from varieties to dosage forms. Sustained-release and controlled-release preparations have always been concerned by people. Under some special pathological conditions, a traditional or single release drug release mode is sometimes difficult to meet the clinical needs. For example, it is difficult for ordinary immediate-release preparations to maintain their efficacy for a long time, while ordinary sustained-release dosage forms cannot take effect quickly. Therefore, people pay more and more attention to biphasic drug delivery systems. The biphasic drug delivery system has two different release phases, which can realize quick/long-acting, pulse or delayed drug release through specific formulation design or process technology according to the special physical and chemical properties and biopharmaceutical properties of drugs, and the circadian rhythm and complexity of disease onset, thus providing more flexible dosing scheme for clinic.

In order to achieve the purpose of biphasic release, people have carried out extensive research to control the drug to release at a predetermined time and at a predetermined speed, such as a double-layer or multi-layer tablet system, a multi-granule system, a film coating system and a timing capsule system. Compared with the conventional common preparation, its formulation and production process are relatively complex, which has higher requirements on industrial production.

### SUMMARY

In the present invention, a biphasic controlled-release preparation is prepared through dosage form design, which can not only take effect quickly *in vivo,* but also maintain stable release, i.e., near zero-order release at a uniform speed for a long time, which can reduce adverse responses and increase patient compliance. Accordingly, the present invention claims the following technical solution:
A biphasic controlled-release preparation is provided, which consists of a drug-containing immediate-release layer (IR), a drug-containing sustained-release layer (SR) and a drug-free blocking layer (BL); wherein
the drug-containing sustained-release layer (SR) is wrapped between the drug-containing immediate-release layer (IR) and the blocking layer (BL); and the drug-containing immediate-release layer (IR) is formed by compressing in a tableting manner and covering the drug-containing sustained-release layer (SR).

Preferably, the biphasic controlled-release preparation is characterized in that the drug-containing immediate-release layer (IR) consists of the following components: an active substance, a filler, an adhesive, a disintegrant, a lubricant and a flow aid.

Preferably, the biphasic controlled-release preparation is characterized in that the drug-containing sustained-release layer (SR) consists of the following components: an active substance, a sustained-release polymer, a filler, an adhesive, a lubricant and a flow aid, wherein the drug-containing sustained-release layer (SR) and the drug-free blocking layer (BL) constitute a partial coating structure with one side opening, wherein the drug-containing sustained-release layer (SR) serves as a tablet core, and the drug-free blocking layer (BL) functions as a partial coating layer. Preferably, the biphasic controlled-release preparation is characterized in that the drug-containing immediate-release layer (IR) consists of the following components: an active substance, a filler, an adhesive, a disintegrant, a lubricant and a flow aid.

Preferably, the biphasic controlled-release preparation is characterized in that the drug-containing sustained-release layer (SR) consists of the following components: an active substance, a sustained-release polymer, a filler, an adhesive, a lubricant and a flow aid; and the drug-containing sustained-release layer is wrapped between the drug-containing immediate-release layer and the blocking layer. Preferably, the biphasic controlled-release preparation is characterized in that the drug-free blocking layer (BL) consists of the following components: a sustained-release polymer, a filler, an adhesive, a lubricant and a flow aid.

Preferably, the biphasic controlled-release preparation described in any one of the above is characterized in that,
a ratio of the mass of the drug-containing immediate-release layer (IR) to the mass of the drug-containing sustained-release layer (SR) is 5:1-1.5:1;
a ratio of the mass of the drug-containing immediate-release layer (IR) to the mass of the drug-free blocking layer (BL) is 1:1-0.125:1; and
a ratio of the mass of the drug-containing sustained-release layer (SR) to the mass of the drug-free blocking layer (BL) is 0.2:1-0.05:1.

Preferably, the biphasic controlled-release preparation is characterized in that the content of the filler in the drug-containing sustained-release layer (SR) is 30-70%, and preferably 50%; and the filler material is a brittle filler, e.g. one or more of lactose, sucrose, calcium sulfate, calcium carbonate and calcium phosphate, and preferably lactose.

Preferably, the biphasic controlled-release preparation is characterized in that the content of an adhesive in the drug-containing immediate-release layer (IR) is 3-10%, the content of an adhesive in the drug-free blocking layer (BL) is 2-10%, and the adhesive is selected from one or more of HPC, HPMC, povidone and sodium carboxymethyl cellulose; wherein the viscosity of HPC is 75-700 cps, and the viscosity of HPMC is 3-50 cps.

Preferably, the biphasic controlled-release preparation is characterized in that the content of the adhesive in the drug-containing immediate-release layer (IR) is 5%, and the material is composed of HPMC and HPC in a content ratio of 0-1:1.

Preferably, the biphasic controlled-release preparation is characterized in that the mass of an active substance in the drug-containing sustained-release layer is greater than that in the drug-containing immediate-release layer, and a ratio of the mass of the active substance in the drug-containing sustained-release layer to the mass of the active substance in the drug-containing immediate-release layer is ≤ 4; and preferably 4:1-1:1, and preferably 2 ≤ ratio ≤ 4.

Preferably, the biphasic controlled-release preparation described in any one of the above is characterized in that a disintegrant in the drug-containing immediate-release layer is selected from one or more of crosslinked sodium carboxymethyl starch, crosslinked sodium carboxymethyl cellulose, crospovidone PVPP and low-substituted hydroxypropyl cellulose L-HPC; and preferably crosslinked sodium carboxymethyl starch or crosslinked sodium carboxymethyl cellulose;
the lubricants in the drug-containing immediate-release layer (IR), the drug-containing sustained-release layer (SR) and the drug-free blocking layer (BL) are independently selected from one or more of magnesium stearate, stearic acid, sodium stearyl fumarate, glyceryl behenate, hydrogenated castor oil and sodium dodecyl sulfate; and preferably magnesium stearate or stearic acid;
the flow aids in the drug-containing immediate-release layer (IR), the drug-containing sustained-release layer (SR) and the drug-free blocking layer (BL) are independently colloidal silicon dioxide or talcum powder; and preferably colloidal silicon dioxide; and
the sustained-release polymer (skeleton material) in the drug-containing sustained-release layer (SR) and the drug-free blocking layer (BL) is hydroxypropyl methylcellulose.

Preferably, the biphasic controlled-release preparation is characterized in that the content of the hydroxypropyl methylcellulose in the drug-containing sustained-release layer (SR) is 20.00-60.00%, and preferably 25.00-50.00%; and
the content of the hydroxypropyl methylcellulose in the drug-free blocking layer (BL) is 20.00-60.00%, and preferably 25.00-50.00%.

Preferably, the biphasic controlled-release preparation is characterized in that the hydroxypropyl methylcellulose in the sustained-release polymer is selected from one or more of E30LV, E50LV, K100LV, K4M, K15M, K100M and xanthan gum.

Preferably, in an *in vitro* dissolution test, at least 85% of active ingredients in the drug-containing immediate-release layer (IR) are dissolved within 45 min, and a trend slope of a dissolution curve of active ingredients in the drug-containing sustained-release layer (SR) within 1-10 hours is between 5.0-5.6, with at least 95% being released within 20 hours.

The innovation of the present invention lies in the tablet shape and structure and the formula of auxiliary materials, which is suitable for any compound active drug that needs to obtain such release mode.

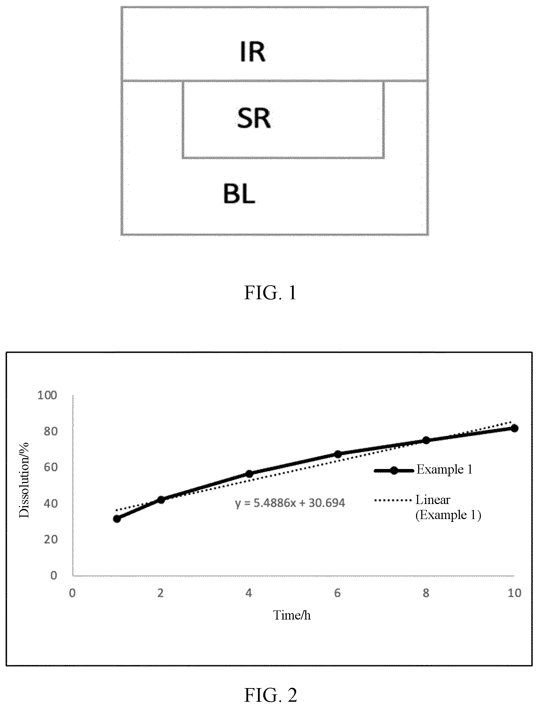

In another aspect of the present invention, provided is a method for preparing the biphasic controlled-release preparation described in any one of the above, which includes the following steps:
a. preparing granules of an immediate-release layer according to a formulation for later use;
b. preparing granules of a sustained-release layer according to a formulation for later use;
c. preparing granules of a blocking layer according to a formulation for later use;
d. placing the granules of the sustained-release layer into a punching die of a tablet press, and compressing the granules into a plain tablet core for later use; and
e. placing a formulation amount of the granules of the blocking layer into a punching die of a tablet press, placing a plain tablet core layer in the center of the granules of the blocking layer, and precompressing at a pre-compression pressure of 0.1-2 KN, so that the plain tablet core forms the drug-containing sustained-release layer (SR), and the plain tablet core is trapped in the granules of the blocking layer, and all faces except the top face of the plain tablet core are surrounded by the blocking layer, thereby forming a partial coating structure with one side opening; and then placing a formulation amount of the granules of the immediate-release layer into the punching die, and applying a main pressure of 5-35 KN, so that the granules of the immediate-release layer cover and adhere on the opening of the partial coating structure in a compression manner to form the drug-containing immediate-release layer (IR), thereby obtaining a finished tablet, the tablet shape and structure of which are as shown in FIG. 1.

Preferably, in the method, the steps a, b and c are specifically as follows:
a. weighing the active substance, the filler, the disintegrant and the adhesive other than HPC in a formulation amount required for preparing the drug-containing immediate-release layer, carrying out wet granulation by addition of purified water or an alcohol solution, sieving wet granules through a 1,000-8,000 µm sieve, drying the wet granules, sieving dry wet granules through a 1,000-8,000 µm sieve, adding a flow aid, premixing, and then adding a lubricant and mixing to prepare granules of the immediate-release layer for later use;
b. weighing the active substance, the sustained-release polymer and the filler in a formulation amount required for preparing the drug-containing sustained-release layer, carrying out wet granulation with purified water or an alcohol solution, sieving wet granules through a 1,000-8,000 µm sieve, drying the wet granules, sieving dry granules through a 1,000-8,000 µm sieve, adding a flow aid, premixing, then adding a lubricant and mixing to prepare granules of the sustained-release layer for later use; and
c. weighing the sustained-release polymer, the filler and the adhesive other than HPC in a formulation amount required for preparing the blocking layer, fully mixing them, carrying out wet granulation by addition of purified water or an alcohol solution, sieving wet granules through a 1,000-8,000 µm sieve, drying the wet granules, sieving dry granules through a 1,000-8,000 µm sieve, adding a flow aid, premixing, then adding a lubricant and mixing to prepare granules of the blocking layer for later use;
wherein in the step e, the pre-compression pressure is 0.1-0.5 KN; and a main pressure is 10-25 KN. Preferably, in the method, when the adhesive materials in the drug-containing immediate-release layer (IR) and the blocking layer include HPC, the HPC is added by being dissolved in the purified water or alcohol solution used for the wet granulation.

The technical advantages of the present invention are summarized as follows:
In the present invention, in order to realize biphasic release of active drugs, that is, to take effect quickly enough and maintain zero-order stable release for a long time, a new tablet shape structure as well as an auxiliary material formula and process for realizing ideal biphasic release of the tablet shape structure are developed. Compared with common preparations, the tablet can achieve the effect of reducing adverse responses and increasing patients' compliance. The immediate-release layer is rapidly dissolved and released in a zero-order mode within 1-10 h, achieving a stable release effect.

### BRIEF DESCRIPTION OF DRAWINGS:

FIG. 1 is a schematic diagram of a tablet shape structure of the present invention;
FIG. 2 shows an *in vitro* dissolution curve and trend line of an escitalopram oxalate biphasic controlled-release preparation as a tablet 1-1 of Example 1 within 1-10 h;
FIG. 3 shows an *in vitro* dissolution curve and trend line of an escitalopram oxalate biphasic controlled-release preparation as a tablet 2-1 of Example 2 within 1-10 h;
FIG. 4 shows an *in vitro* dissolution curve and trend line of an escitalopram oxalate biphasic controlled-release preparation as a tablet 2-2 of Example 2 within 1-10 h;
FIG. 5 shows an *in vitro* dissolution curve and trend line of an escitalopram oxalate biphasic controlled-release preparation as a tablet 2-3 of Example 2 within 1-10 h;
FIG. 6 shows an *in vitro* dissolution curve and trend line of an escitalopram oxalate biphasic controlled-release preparation as a tablet 3-1 of Example 3 within 1-10 h;
FIG. 7 shows an *in vitro* dissolution curve and trend line of an escitalopram oxalate biphasic controlled-release preparation as a tablet 4-1 of Example 4 within 1-10 h;
FIG. 8 shows an *in vitro* dissolution curve and trend line of an escitalopram oxalate biphasic controlled-release preparation of Example 5 within 1-10 h;
FIG. 9 shows an *in vitro* dissolution curve and trend line of an escitalopram oxalate biphasic controlled-release preparation of Example 6 within 1-10 h;
FIG. 10 shows an *in vitro* dissolution curve and trend line of an escitalopram oxalate biphasic controlled-release preparation of Example 7 within 1-10 h;
FIG. 11 shows an *in vitro* dissolution curve and trend line of an escitalopram oxalate biphasic controlled-release preparation of Comparative Example 1 within 1-10 h; and
FIG. 12 shows *in vitro* dissolution data of tablets with qualified friability within 0-20 hours.

### DETAILED DESCRIPTION

Hereinafter, the technical solution of the present invention will be exemplified by specific embodiments, and it can be understood by those skilled in the art that these embodiments should not be interpreted as limiting the claimed scope of the present invention.

The methods and conditions for dissolution used in the following examples and comparative examples are as follows: dissolution determination method II in China Pharmacopoeia, at 50 rpm, with a medium of 900 ml, at pH 1.2, and at a medium temperature of 37 ± 0.5°C.

For the dissolution curves of the following examples and comparative examples hereafter, the trend line of the dissolution curve within 1-10 h was selected for evaluating a zero-order release rate. When the slope of the trend line of the dissolution curve is between 5.0-5.6, the zero-order release effect is relatively ideal, and can be maintained continuously, so that the drug is completely absorbed. However, when the slope of the dissolution curve is too small (less than 5.0), it indicates that the release rate of the drug in the sustained-release portion is too slow, which will lead to incomplete absorption of the drug and affect the bioavailability of the drug. When the slope of the dissolution curve is too large (greater than 5.6), it indicates that the release rate of the drug in the sustained-release portion is too fast, which will lead to the inability to maintain the approximate zero-order release of drug in the later stage and affect the sustained-release effect of the drug.

### Example 1 Preparation of 1,000 tablets of biphasic controlled-release preparation

Taking the preparation of 1,000 escitalopram oxalate controlled-release tablets as an example, the composition and weight of each component of the tablet were as shown in the table below:

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | Microcrystalline cellulose MCC-PH101 | 134.75 |
| | Adhesive | HPMC K100LV | 5 |
| | Adhesive | Povidone | 7.5 |
| | Flow aid | Silicon dioxide | 0.625 |
| | Lubricant | Sodium stearyl fumarate | 0.625 |
| | **Total amount of immediate-release layer** | | 250 |
| **Sustained-release layer** | Active drug | Escitalopram oxalate | 9.588 |
| | Sustained-release polymer | HPMC K100M | 18 |
| | Filler | Sucrose | 31.212 |
| | Flow aid | Silicon dioxide | 0.6 |
| | Lubricant | Sodium stearyl fumarate | 0.6 |
| | **Total amount of sustained-release layer** | | 60 |
| **Blocking layer** | Sustained-release polymer | HPMC K4M | 260 |
| | Sustained-release polymer | HPMC K15M | 65 |
| | Filler | Corn starch | 97.5 |
| | Filler | MCC-PH101 | 191.75 |
| | Adhesive | HPC (external addition) | 32.5 |
| | Flow aid | Silicon dioxide | 1.625 |
| | Lubricant | Sodium stearyl fumarate | 1.625 |
| | **Total amount of blocking layer** | | 650 |

The method for preparing escitalopram oxalate biphasic controlled-release preparation in Example 1 of the present invention included the following steps:
a. preparation of a drug-containing immediate-release layer (IR): an active drug, a disintegrant, a filler and an adhesive were weighed according to a formulation, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with a flow aid, premixed, then added with a lubricant, and mixed to prepare granules of an immediate-release layer for later use.
b. preparation of a drug-containing sustained-release layer (SR): an active drug, a sustained-release polymer and a filler were weighed according to a formulation, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with a flow aid, premixed, then added with a lubricant, and mixed to prepare granules of a sustained-release layer for later use.
c. preparation of a blocking layer (BL): a sustained-release polymer and a filler were weighed according to a formulation, mixed fully, and added with an adhesive HPC dissolved in purified water or an alcohol solution for wet granulation. Wet granules were sieved through a 1,000-8,000 µm sieve, and dried. Dry granules were sieved through a 1,000-8,000 µm sieve, added with a flow aid, premixed, then added with a lubricant, and mixed to prepare granules of a blocking layer for later use.
d. a formulation amount of the granules of the drug-containing sustained-release layer was placed into a punching die of a tablet press, and compressed into a plain tablet core for later use;
e. a formulation amount of the granules of the blocking layer was placed into a punching die of a tablet press, a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer, thereby forming a partial coating structure with one side opening; and then a formulation amount of the granules of the immediate-release layer was placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 1-1.

The tablet shape structure of the biphasic controlled-release preparation of the present invention was as shown in FIG. 1, and the subsequent examples were the same.

The appearance, tablet cracking condition, friability and dissolution of tablet 1-1 in Example 1 were investigated. The experimental data was as shown in the table below:

| | Appearance | Tablet cracking condition | Number of cracked tablets/100 tablets | Friability (%) | Is the friability qualified |
|---|---|---|---|---|---|
| Tablet 1-1 | Qualified | No tablet cracking | 0 | 0.12 | Qualified |

It could be seen from this that, the tablet 1-1 was qualified in terms of appearance and friability. The tablet 1-1 was subjected to an *in vitro* dissolution experiment. The dissolution curve and trend line were as shown in FIG. 2, and the zero-order release rate (trend line slope) was 5.49.

### Example 2 Preparation of escitalopram oxalate biphasic controlled-release preparation, and investigation of the effects of different IR:SR ratios

Escitalopram oxalate controlled-release tablets were prepared, and the composition and weight of each component of the tablet were as shown in the table below:

| | | | Tablet 2-1 |
|---|---|---|---|
| Tablet layer | Function | Material | Unit Formulation amount (mg) |
| Immediate-release layer | Active drug | Escitalopram oxalate | 6.4 |
| | Disintegrant | Crosslinked sodium carboxylmethyl cellulose | 12 |
| | Filler | Corn starch | 144 |
| | Filler | MCC-PH101 | 203.6 |
| | Adhesive | Sodium carboxymethyl cellulose | 32 |
| | Flow aid | Talc powder | 1 |
| | Lubricant | Stearic acid | 1 |
| | | Total amount of immediate-release layer | 400 |
| Sustained-release layer | Active drug | Escitalopram oxalate | 12.784 |
| | Skeletal material | HPMC K100LV | 32 |
| | Filler | Lactose GranuLac 200 | 0 |
| | Flow aid | Talc powder | 1.375 |
| | Lubricant | Stearic acid | 1.5675 |
| | | Total amount of sustained- | 80 |
| | | release layer | |
| Blocking layer | Skeletal material | HPMC K100LV | 220 |
| | Filler | Pregelatinized starch | 137.5 |
| | Filler | MCC-PH101 | 156.75 |
| | Adhesive | HPC (external addition) | 33 |
| | Flow aid | Talc powder | 1.375 |
| | Lubricant | Stearic acid | 1.375 |
| | | Total amount of blocking layer | 650 |

The method for preparing escitalopram oxalate biphasic controlled-release preparation in Example 2 of the present invention included the following steps:
a. preparation of a drug-containing immediate-release layer: escitalopram oxalate, corn starch, microcrystalline cellulose, crosslinked sodium carboxymethyl cellulose and sodium carboxymethyl cellulose were weighed, and mixed fully. Wet granulation was conducted by addition of purified water or an alcohol solution. Wet granules were sieved through a 1,000-8,000 µm sieve, and dried. Dry granules were sieved through a 1,000-8,000 µm sieve, added with talcum powder, premixed, then added with stearic acid, and mixed to prepare granules of the immediate-release layer for later use.
b. preparation of a drug-containing sustained-release layer: escitalopram oxalate, HPMC K100LV and lactose were weighed, and mixed fully, and subjected to wet granulation by addition of purified water or an alcohol solution, Wet granules were sieved through a 1,000-8,000 µm sieve, and dried. Dry granules were sieved through a 1,000-8,000 µm sieve, added with talcum powder, premixed, then added with 0 stearic acid and mixed to prepare granules of the sustained-release layer for later use.
c. preparation of a blocking layer: the required formulation amount of HPMCK4LV, pregelatinized starch and microcrystalline cellulose were weighed, mixed fully, and added with HPC-GF and dissolved in purified water or an alcohol solution for wet granulation. Wet granules were sieved through a 1,000-8,000 µm sieve, and dried. dry granules were sieved through a 1,000-8,000 µm sieve, added with 1 talcum powder, premixed, then added with stearic acid and mixed to prepare the granules of the blocking layer for later use.
d. a formulation amount of the granules of the drug-containing sustained-release layer was placed into a punching die of a tablet press, and respectively compressed into plain tablet cores of 40 mg, 50 mg and 80 mg for later use;
e. 650 mg of the granules of the blocking layer were placed into a punching die of a tablet press, 50 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer, thereby forming a partial coating structure with one side opening; and then 400 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 2-1.

650 mg of the granules of the blocking layer were placed into a punching die of a tablet press, 80 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer, thereby forming a partial coating structure with one side opening; and then 100 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 2-2.

650 mg of the granules of the blocking layer were placed into a punching die of a tablet press, 40 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer, thereby forming a partial coating structure with one side opening; and then 220 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 2-3.

The appearance, tablet cracking condition, friability and dissolution of tablets 2-1, 2-2 and 2-3 in Comparative Example 2 were investigated. The experimental data was as shown in the table below:

| | Appearance | Tablet cracking condition | Number of cracked tablets/100 tablets | Friability (%) | Is the friability qualified |
|---|---|---|---|---|---|
| Tablet 2-1 | Qualified | No tablet cracking | 0 | 0.18 | Qualified |
| Tablet 2-2 | Qualified | No tablet cracking | 0 | 0.13 | Qualified |
| Tablet 2-3 | Qualified | No tablet cracking | 0 | 0.09 | Qualified |

It could be seen from the above that tablets 2-1, 2-2 and 2-3 were qualified in terms of appearance and friability under the condition of different weight ratios of immediate-release and sustained-release layers.

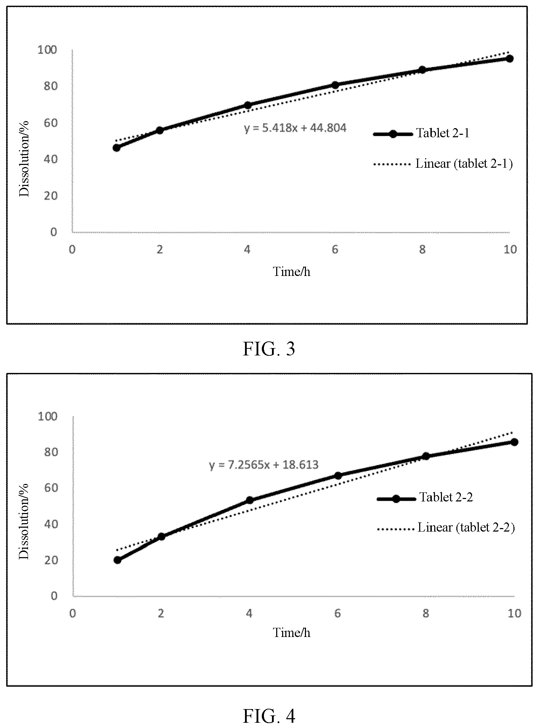

The tablets 2-1, 2-2 and 2-3 were subjected to *in vitro* dissolution experiments, and the dissolution curves and trend charts were as shown in FIGs. 3, 4 and 5, respectively. By comparing the release rates of tablets 2-1, 2-2 and 2-3 within 1-10 h, it could be seen that the zero-order release rates (trend line slopes) of the tablets were 5.42, 7.25 and 5.73, respectively; and the dissolution rates of the tablets 2-2 and 2-3 was faster.

### Example 3 Preparation of escitalopram oxalate biphasic controlled-release preparation (investigation of different SR:BL ratios)

Granules of different tablet layers were prepared according to the formulation and process of Example 2.

800 mg of the granules of the blocking layer were placed into a punching die of a tablet press, 40 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then 200 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 3-1, i.e., IR:SR = 5:1, IR:BL = 1:4, and SR:BL = 0.05:1.

300 mg of the granules of the blocking layer were placed into a punching die of a tablet press, 80 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then 200 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 3-2, i.e., IR:SR = 2.5:1, IR:BL = 0.67: 1, and SR:BL = 0.27:1.

The appearance, tablet cracking condition, friability and *in vitro* dissolution of tablets 3-1 and 3-2 in Comparative Example 3 were investigated. The experimental data was as shown in the table below:

| | Appearance | Tablet cracking condition | Number of cracked tablets/100 tablets | Friability (%) | Is the friability qualified |
|---|---|---|---|---|---|
| Tablet 3-1 | Qualified | No tablet cracking | 0 | 0.17 | Qualified |
| Tablet 3-2 | Unqualified | Tablet cracking | 87 | 1.23 | Unqualified |

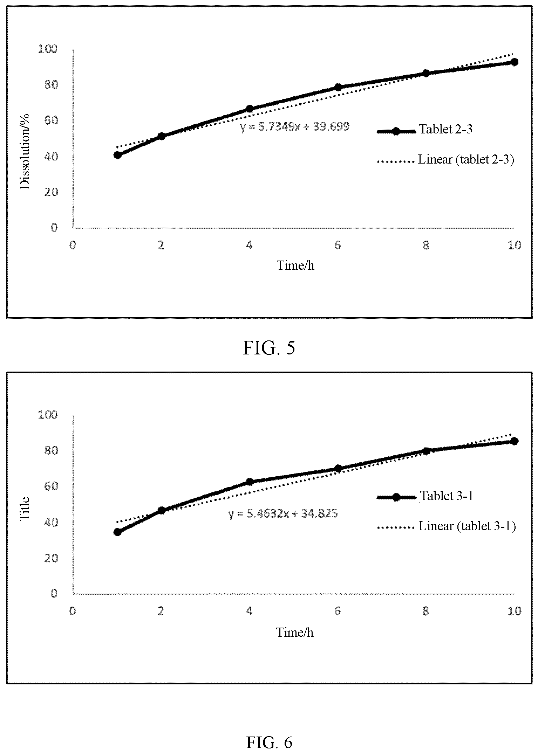

It could be seen from the above that, under the condition of different weight ratios of the sustained-release and blocking layers, tablet 3-1 was qualified in terms of both appearance and friability, and the tablet 3-2 had a tablet cracking phenomenon and was unqualified in terms of friability. The tablet 3-1 was subjected to an *in vitro* dissolution experiment. The dissolution curve and trend line were as shown in FIG. 6, and the zero-order release rate (trend line slope) was 5.46.

### Example 4 Preparation of escitalopram oxalate biphasic controlled-release preparation (investigation of different IR:BL ratios)

Granules of different tablet layers were prepared according to the formulation and process of Example 2.

200 mg of the granules of the blocking layer were placed into a punching die of a tablet press, 40 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then 200 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 4-1, i.e. IR:SR = 5:1, IR:BL = 1:1, and SR:BL = 0.2:1.

200 mg of the granules of the blocking layer were placed into a punching die of a tablet press, 40 mg of a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then 300 mg of the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet 4-2, i.e. IR:SR = 7.5:1, IR:BL = 1.5:1, and SR:BL = 0.2:1.

The appearance, tablet cracking condition, friability and *in vitro* dissolution of tablets 4-1 and 4-2 in Comparative Example 4 were investigated. The experimental data was as shown in the table below:

| | Appearance | Tablet cracking condition | Number of cracked tablets/100 tablets | Friability (%) | Is the friability qualified |
|---|---|---|---|---|---|
| Tablet 4-1 | Qualified | No tablet cracking | 0 | 0.05 | Qualified |
| Tablet 4-2 | Unqualified | Tablet cracking | 82 | 1.23 | Unqualified |

It could be seen from the above that, under the condition of different weight ratios of the immediate-release and blocking layers, the tablet 4-1 was qualified in terms of both the appearance and friability, and the tablet 4-2 was cracked during compression due to the relatively small mass of the BL layer, and was unqualified in terms of both the appearance and friability.

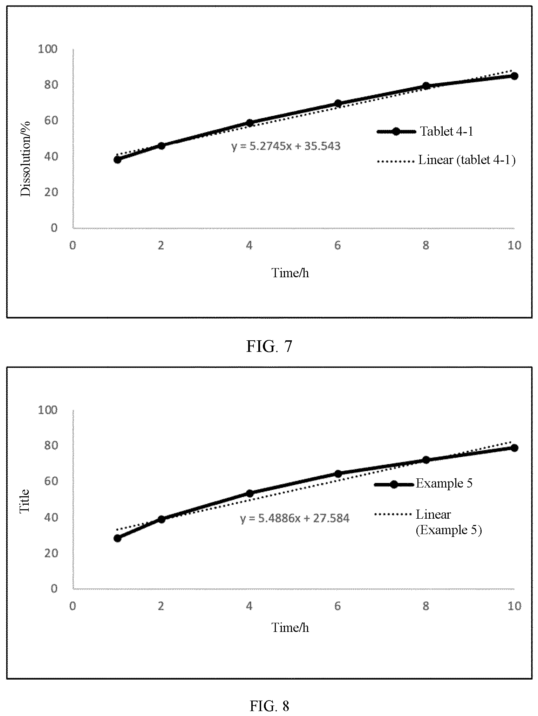

The tablet 4-1 was subjected to an *in vitro* dissolution experiment. The dissolution curve and trend line were as shown in FIG. 7, and the zero-order release rate (trend line slope) was 5.27.

**The following Examples 5-7 and Comparative Example 3 investigated the proportion of adhesive materials in the immediate-release IR layer.**

### Example 5 Preparation of 1,000 tablets of biphasic controlled-release preparation (HPMC 0%, HPC 5%, i.e. 0:5)

Taking the preparation of 1,000 escitalopram oxalate controlled-release tablets as an example, the composition and weight of each component of the tablet were as shown in the table below:

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | MCC-PH101 | 134.75 |
| | Adhesive | **HPMC K100LV** | **0** |
| | Adhesive | **HPC (external addition)** | **12.5** |
| | Flow aid | Silicon dioxide | 0.625 |
| | Lubricant | Sodium stearyl fumarate | 0.625 |
| | Total amount of immediate-release layer | | 250 |
| **Sustained-release layer** | Active drug | Escitalopram oxalate | 9.588 |
| | Sustained-release polymer | HPMC K100M | 18 |
| | Filler | Sucrose | 31.212 |
| | Flow aid | Silicon dioxide | 0.6 |
| | Lubricant | Sodium stearyl fumarate | 0.6 |
| | Total amount of sustained-release layer | | 60 |
| **Blocking layer** | Sustained-release polymer | HPMC K4M | 260 |
| | Sustained-release polymer | HPMC K15M | 65 |
| | Filler | Corn starch | 97.5 |
| | Filler | MCC-PH101 | 191.75 |
| | Adhesive | HPC (external addition) | 32.5 |
| | Flow aid | Silicon dioxide | 1.625 |
| | Lubricant | Sodium stearyl fumarate | 1.625 |
| | Total amount of blocking layer | | 650 |

The preparation method was as follows:
a. preparation of a drug-containing immediate-release layer: escitalopram oxalate, crospovidone PVPP, pregelatinized starch, pregelatinized starch and HPMC K100LV were weighed, fully mixed, and added with HPC-GF dissolved with purified water or an alcohol solution for wet granulation. Wet granules were sieved through a 1,000-8,000 µm sieve, and dried. Dry granules were sieved through a 1,000-8,000 µm sieve, added with silicon dioxide, premixed, then added with sodium stearyl fumarate, and mixed to prepare granules of the immediate-release layer for later use.
b. preparation of a drug-containing sustained-release layer: escitalopram oxalate, HPMC K100M and sucrose were weighed, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with silicon dioxide, premixed, then added with sodium stearyl fumarate and mixed to prepare granules of the sustained-release layer for later use.
c. preparation of a blocking layer: the required formulation amount of HPMC K4M, HPMC K15M, corn starch and microcrystalline cellulose MCC-PH101 were weighed, mixed fully, and added with HPC-GF dissolved in purified water or an alcohol solution for wet granulation. Wet granules were sieved through a 1,000-8,000 µm sieve, and dried. Dry granules were sieved through a 1,000-8,000 µm sieve, added with silicon dioxide, premixed, then added with sodium stearyl fumarate and mixed to prepare granules of the blocking layer for later use.
d. a formulation amount of the granules of the drug-containing sustained-release layer was placed into a punching die of a tablet press, and compressed into a plain tablet core for later use;
e. the granules of the blocking layer were placed into a punching die of a tablet press, a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer, thereby forming a partial coating structure with one side opening; and then the granules of the immediate-release layer were placed into the punching die, and a main pressure (20 KN) was applied, so that the granules of the immediate-release layer covered and adhered on the opening of the partial coating structure in a compression manner to form the drug-containing immediate-release layer (IR), thereby obtaining finished tablets respectively.

**Example 6 Preparation of 1,000 tablets of biphasic controlled-release preparation (HPMC 1%, HPC 4%, i.e. 1:4)** Taking the preparation of 1,000 escitalopram oxalate controlled-release tablets as an example, the composition and weight of each component of the tablet were as shown in the table below:

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | MCC-PH101 | 134.75 |
| | Adhesive | HPMC K100LV | 2.5 |
| | Adhesive | HPC (external addition) | 10 |
| | Flow aid | Silicon dioxide | 0.625 |
| | Lubricant | Sodium stearyl fumarate | 0.625 |
| | Total amount of immediate-release layer | | 250 |
| **Sustained-release layer** | Active drug | Escitalopram oxalate | 9.588 |
| | Sustained-release polymer | HPMC K100M | 18 |
| | Filler | Sucrose | 31.212 |
| | Flow aid | Silicon dioxide | 0.6 |
| | Lubricant | Sodium stearyl fumarate | 0.6 |
| | Total amount of sustained-release layer | | 60 |
| **Blocking layer** | Sustained-release polymer | HPMC K4M | 260 |
| | Sustained-release polymer | HPMC K15M | 65 |
| | Filler | Corn starch | 97.5 |
| | Filler | MCC-PH101 | 191.75 |
| | Adhesive | HPC (external addition) | 32.5 |
| | Flow aid | Silicon dioxide | 1.625 |
| | Lubricant | Sodium stearyl fumarate | 1.625 |
| | Total amount of blocking layer | | 650 |

The preparation method was the same as that of Example 5.

### Example 7 Preparation of 1,000 tablets of biphasic controlled-release preparation (HPMC 2%, HPC 3%, i.e. 2:3)

Taking the preparation of 1,000 escitalopram oxalate controlled-release tablets as an example, the composition and weight of each component of the tablet were as shown in the table below:

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | MCC-PH101 | 134.75 |
| | Adhesive | HPMC K100LV | 5 |
| | Adhesive | HPC-GF (external addition) | 7.5 |
| | Flow aid | Silicon dioxide | 0.625 |
| | Lubricant | Sodium stearyl fumarate | 0.625 |
| | Total amount of immediate-release layer | | 250 |
| **Sustained-release layer** | Active drug | Escitalopram oxalate | 9.588 |
| | Sustained-release polymer | HPMC K100M | 18 |
| | Filler | Sucrose | 31.212 |
| | Flow aid | Silicon dioxide | 0.6 |
| | Lubricant | Sodium stearyl fumarate | 0.6 |
| | Total amount of sustained-release layer | | 60 |
| **Blocking layer** | Sustained-release polymer | HPMC K4M | 260 |
| | Sustained-release polymer | HPMC K15M | 65 |
| | Filler | Corn starch | 97.5 |
| | Filler | MCC-PH101 | 191.75 |
| | Adhesive | HPC-EF (external addition) | 32.5 |
| | Flow aid | Silicon dioxide | 1.625 |
| | Lubricant | Sodium stearyl fumarate | 1.625 |
| | Total amount of blocking layer | | 650 |

The preparation method was the same as that of Example 5.

### Comparative Example 1 Preparation of 1,000 tablets of biphasic controlled-release preparation (investigation of the content of an IR adhesive, with the total amount exceeding 10%)

Taking the preparation of 1,000 escitalopram oxalate controlled-release tablets as an example, the composition and weight of each component of the tablet were as shown in the table below:

| Tablet layer | Function | Formulation | Mass (g) |
|---|---|---|---|
| Immediate-release layer | Active drug | Escitalopram oxalate | 4 |
| | Disintegrant | Crospovidone PVPP | 7.5 |
| | Filler | Pregelatinized starch | 90 |
| | Filler | MCC-PH101 | 119.75 |
| | Adhesive | HPC | 12.5 |
| | Adhesive | Povidone | 15 |
| | Flow aid | Silicon dioxide | 0.625 |
| | Lubricant | Sodium stearyl fumarate | 0.625 |
| | **Mass of immediate-release layer** | | 250 |
| Sustained-release layer | Drug | Escitalopram oxalate | 9.588 |
| | Sustained-release polymer (skeletal material) | HPMC K100M | 18 |
| | Filler | Sucrose | 31.212 |
| | Flow aid | Silicon dioxide | 0.6 |
| | Lubricant | Sodium stearyl fumarate | 0.6 |
| | **Mass of sustained-release layer** | | 60 |
| Blocking layer | Sustained-release polymer (skeletal material) | HPMC K4M | 260 |
| | Sustained-release polymer (skeletal material) | HPMC K15M | 65 |
| | Filler | Corn starch | 97.5 |
| | Filler | MCC-PH101 | 172.25 |
| | Adhesive | HPC (external addition) | 52 |
| | Flow aid | Silicon dioxide | 1.625 |
| | Lubricant | Sodium stearyl fumarate | 1.625 |
| | **Mass of blocking layer** | | 650 |

The preparation method was the same as that of Example 5.

The method for preparing escitalopram oxalate biphasic controlled-release preparation in the Comparative Example of the present invention included the following steps:
a. preparation of a drug-containing immediate-release layer: 4 g of escitalopram oxalate, 90 g of pregelatinized starch, 119.75 g of microcrystalline cellulose, 7.5 g of crospovidone PVPP, 12.5 g of HPMC-HF and 7.5 g of povidone were weighed, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with 0.625 g of silicon dioxide, premixed, then added with 0.625 g of sodium stearyl fumarate, and mixed to prepare granules of an immediate-release layer for later use.
b. preparation of a drug-containing sustained-release layer: 9.588 g of escitalopram oxalate, 18 g of HPMC K100M and 31.212 g of sucrose were weighed, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with 0.6 g of silicon dioxide, premixed, then added with 0.6 g of sodium stearyl fumarate, and mixed to prepare granules of a sustained-release layer for later use.
c. preparation of a blocking layer: the required formulation amount, i.e., 260 g of HPMCK4M, 65 g of HPMCK15M, 97.5 g of corn starch and 172.25 g of microcrystalline cellulose were weighed, mixed fully, and added with 52 g of HPC dissolved in purified water or an alcohol solution for wet granulation. Wet granules were sieved through a 1,000-8,000 µm sieve, and dried. Dry granules were sieved through a 1,000-8,000 µm sieve, added with 1.625 g of silicon dioxide, premixed, then added with 1.625 g of sodium stearyl fumarate, and mixed to prepare granules of the blocking layer for later use.
d. a formulation amount of the granules of the drug-containing sustained-release layer was placed into a punching die of a tablet press, and compressed into a plain tablet core for later use;
e. a formulation amount of the granules of the blocking layer was placed into a punching die of a tablet press, a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then a formulation amount of the granules of the immediate-release layer was placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet.

The appearance, tablet cracking condition, friability and dissolution of the biphasic controlled-release preparation compressed in Comparative Example 1 were investigated. The experimental data was as shown in the table below:

| | Appearance | Tablet cracking condition | Number of cracked tablets/100 tablets | Friability (%) | Is the friability qualified |
|---|---|---|---|---|---|
| Comparative Example 1 | Pitted surface and rough | No tablet cracking | 0 | 0.38 | Qualified |

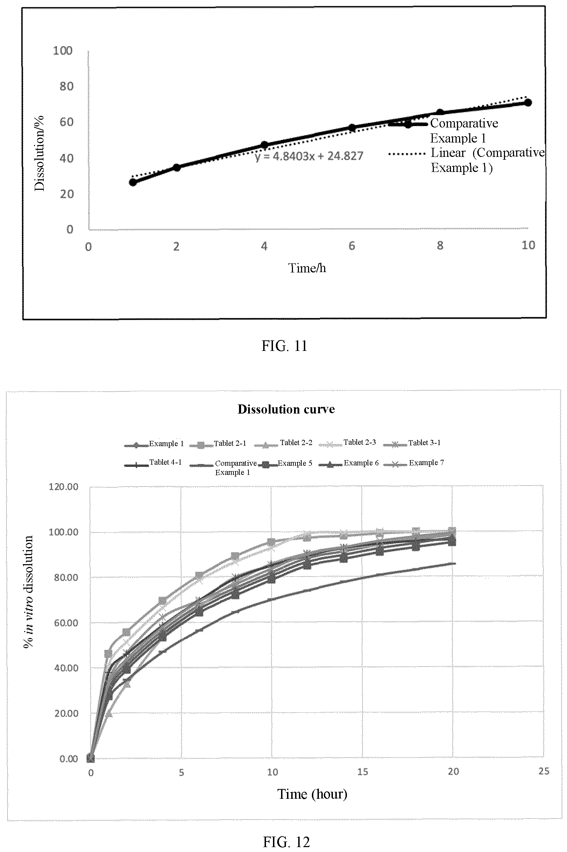

It could be seen from the above that the compressed biphasic controlled-release preparation of Comparative Example 1 had no tablet cracking, and was qualified in terms of friability, but the appearance was rough. The compressed biphasic controlled-release preparation of Comparative Example 1 was subjected to an *in vitro* dissolution experiment, and the dissolution curve was as shown in FIG. 11.

By comparing its release rate within 1-10 h, it could be seen that the dissolution rate of the biphasic controlled-release preparation of Comparative Example 1 was too slow *in vitro* within 1-10 h, and the zero-order release rate (trend line slope) was 4.84, which did not reach the ideal release mode.

### Comparative Example 2 Preparation of 1,000 tablets of biphasic controlled-release preparation (investigation of the selection of SR filler materials)

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | MCC-PH101 | 134.75 |
| | Adhesive | HPMCK100LV | 5 |
| | Adhesive | Povidone | 7.5 |
| | Flow aid | Silicon dioxide | 0.625 |
| | Lubricant | Sodium stearyl fumarate | 0.625 |
| | | **Mass of immediate-release layer** | 250 |
| **Sustained-release layer** | Drug | Escitalopram oxalate | 9.588 |
| | Sustained-release polymer (skeletal material) | HPMC K100M | 18 |
| | Filler | Microcrystalline cellulose | 31.212 |
| | Flow aid | Silicon dioxide | 0.6 |
| | Lubricant | Sodium stearyl fumarate | 0.6 |
| | | **Mass of sustained-release layer** | 60 |
| **Blocking layer** | Sustained-release polymer (skeletal material) | HPMC K4M | 260 |
| | Sustained-release polymer (skeletal material) | HPMC K15M | 65 |
| | Filler | Corn starch | 97.5 |
| | Filler | MCC-PH101 | 191.75 |
| | Adhesive | HPC (external addition) | 32.5 |
| | Flow aid | Silicon dioxide | 1.625 |
| | Lubricant | Sodium stearyl fumarate | 1.625 |
| | | **Mass of blocking layer** | 650 |

The preparation method was the same as that of Example 1.

The method for preparing escitalopram oxalate biphasic controlled-release preparation in Comparative Example 2 of the present invention included the following steps:
a. preparation of a drug-containing immediate-release layer: 4 g of escitalopram oxalate, 90 g of pregelatinized starch, 134.75 g of microcrystalline cellulose, 7.5 g of crospovidone PVPP, 5 g of HPMC K4M and 7.5 g of povidone were weighed, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with 0.625 g of silicon dioxide, premixed, then added with 0.625 g of sodium stearyl fumarate, and mixed to prepare granules of an immediate-release layer for later use.
b. preparation of a drug-containing sustained-release layer: 9.588 g of escitalopram oxalate, 18 g of HPMC K100M and 31.212 g of microcrystalline cellulose were weighed, mixed fully, and subjected to wet granulation by addition of purified water or an alcohol solution. Wet granules were sieved through a 1,000-8,000 µm sieve, and dried. Dry granules were sieved through a 1,000-8,000 µm sieve, added with 0.6 g of silicon dioxide, premixed, then added with 0.6 g of sodium stearyl fumarate, and mixed to prepare granules of a sustained-release layer for later use.
c. preparation of a blocking layer: the required formulation amount, i.e., 260 g of HPMCK4M, 65 g of HPMCK15M, 97.5 g of corn starch and 191.75 g of microcrystalline cellulose were weighed, mixed fully, and added with 32.5 g of HPC dissolved in purified water or an alcohol solution for wet granulation. Wet granules were sieved through a 1,000-8,000 µm sieve, and dried. Dry granules were sieved through a 1,000-8,000 µm sieve, added with 1.625 g of silicon dioxide, premixed, then added with 1.625 g of sodium stearyl fumarate, and mixed to prepare granules of the blocking layer for later use.
d. a formulation amount of the granules of the drug-containing sustained-release layer was placed into a punching die of a tablet press, and compressed into a plain tablet core for later use;
e. a formulation amount of the granules of the blocking layer was placed into a punching die of a tablet press, a plain tablet core layer was placed in the center of the granules of the blocking layer, and pre-compression was conducted (1 KN), so that the plain tablet core formed the drug-containing sustained-release layer (SR), and the plain tablet core was trapped in the granules of the blocking layer, and all faces except the top face of the plain tablet core were surrounded by the blocking layer (BL), thereby forming a partial coating structure with one side opening; and then a formulationed amount of the granules of the immediate-release layer was placed into the punching die, and a main pressure (20 KN) was applied, so that the granules of the immediate-release layer covered and adhered on the opening of the partial coating structure in a compression manner to form the drug-containing immediate-release layer (IR), thereby obtaining a finished tablet.

The appearance, tablet cracking condition, friability and dissolution of the biphasic controlled-release preparation compressed in Comparative Example 2 were investigated. The experimental data was as shown in the table below:

| | Appearance | Tablet cracking condition | Number of cracked tablets/100 tablets | Friability (%) | Is the friability qualified |
|---|---|---|---|---|---|
| Comparative Example 2 | Unqualified | Tablet cracking | 95 | 1.33 | Unqualified |

It could be seen from the above that the cracking phenomenon occurred in the process of compression of Comparative Example 2, because microcrystalline cellulose was a non-brittle filler, which was prone to viscoelastic deformation after being compressed, resulting in the immediate-release layer being pushed open, so the friability and appearance were unqualified.

According to the experimental data in comprehensive research and development, it was found that a brittle filler that was not easy to deform, e.g. lactose, sucrose, calcium sulfate, calcium carbonate and calcium phosphate, must be selected for the tablet shape structure of the present invention.

**Comparative Example 3 Preparation of 1,000 tablets of biphasic controlled-release preparation (HPMC 3%, HPC 2%, i.e. 3:2)** Taking the preparation of 1,000 escitalopram oxalate controlled-release tablets as an example, the composition and weight of each component of the tablet were as shown in the table below:

| **Tablet layer** | **Function** | **Formulation** | **Mass (g)** |
|---|---|---|---|
| **Immediate-release layer** | Active drug | Escitalopram oxalate | 4 |
| | Disintegrant | Crospovidone PVPP | 7.5 |
| | Filler | Pregelatinized starch | 90 |
| | Filler | MCC-PH101 | 134.75 |
| | Adhesive | **HPMC K100LV** | **7.5** |
| | Adhesive | **HPC (external addition)** | **5** |
| | Flow aid | Silicon dioxide | 0.625 |
| | Lubricant | Sodium stearyl fumarate | 0.625 |
| | Total amount of immediate-release layer | | 250 |
| **Sustained-release layer** | Active drug | Escitalopram oxalate | 9.588 |
| | Sustained-release | HPMC K100M | 18 |
| | polymer | | |
| | Filler | Sucrose | 31.212 |
| | Flow aid | Silicon dioxide | 0.6 |
| | Lubricant | Sodium stearyl fumarate | 0.6 |
| | | Total amount of sustained-release layer | 60 |
| **Blocking layer** | Sustained-release polymer | HPMC K4M | 260 |
| | Sustained-release polymer | HPMC K15M | 65 |
| | Filler | Corn starch | 97.5 |
| | Filler | MCC-PH101 | 191.75 |
| | Adhesive | HPC-EF (external addition) | 32.5 |
| | Flow aid | Silicon dioxide | 1.625 |
| | Lubricant | Sodium stearyl fumarate | 1.625 |
| | | Total amount of blocking layer | 650 |

The preparation method was the same as that of Example 5.

The appearance, tablet cracking condition, friability and *in vitro* dissolution of Examples 5, 6 and 7 and Comparative Example 3 were investigated. The experimental data was shown in the table below.

| | Appearance | Tablet cracking condition | Number of cracked tablets/100 tablets | Friability (%) | Is the friability qualified |
|---|---|---|---|---|---|
| Example 5 | Qualified | No tablet cracking | 0 | 0.05 | Qualified |
| Example 6 | Qualified | No tablet cracking | 0 | 0.22 | Qualified |
| Example 7 | Qualified | No tablet cracking | 0 | 0.83 | Qualified |
| Comparative Example 3 | Pitted surface Rough | No tablet cracking | 0 | 1.15 | Unqualified |

Through the comparison of the aforementioned data, it could be seen that when the adhesive HPMC:HPC of the immediate-release layer was greater than 1 (Comparative Example 3), the tablet had a rough appearance and a pitted surface, and was unqualified in terms of the friability.

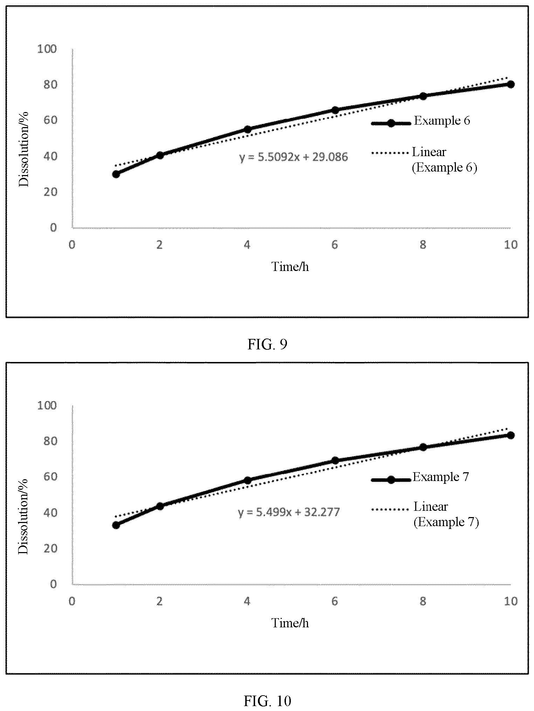

The biphasic controlled-release preparations compressed in Examples 5-7 were subjected to *in vitro* dissolution experiments. The dissolution curves and trend lines were as shown in FIGs. 8-10, and the zero-order release rates (trend line slopes) were 5.49, 5.51 and 5.5, respectively. It could be seen that the friability and *in vitro* dissolution both could reach an ideal state.

Statistics of % *in vitro* dissolution of tablets obtained in Examples 1-7 with qualified non-cracking friability in the tableting process at each time point within 0-20 hours were shown in the table below, and the dissolution curve was as shown in FIG. 12.

| **Time** | **Example 1** | **Tablet 2-1** | **Tablet 2-2** | **Tablet 2-3** | **Tablet 3-1** | **Tablet 4-1** | **Comparative Example 1** | **Example 5** | **Example 6** | **Example 7** |
|---|---|---|---|---|---|---|---|---|---|---|
| 0 | 0.00 | 0.00 | 0.00 | 0.00 | 0.00 | 0.00 | 0.00 | 0.00 | 0.00 | 0.00 |
| 1 | 31.47 | 46.27 | 20.03 | 40.70 | 34.46 | 38.12 | 26.34 | 28.36 | 29.91 | 33.12 |
| 2 | 42.10 | 55.83 | 32.82 | 51.19 | 46.49 | 45.91 | 34.59 | 38.99 | 40.51 | 43.66 |
| 4 | 56.46 | 69.59 | 53.21 | 66.52 | 62.46 | 58.76 | 46.93 | 53.35 | 54.93 | 58.02 |
| 6 | 67.47 | 80.70 | 66.99 | 78.49 | 69.91 | 69.67 | 56.56 | 64.36 | 65.92 | 69.17 |
| 8 | 74.97 | 89.05 | 77.78 | 86.43 | 79.85 | 79.29 | 64.58 | 71.86 | 73.64 | 76.64 |
| 10 | 81.84 | 95.34 | 85.80 | 92.66 | 85.14 | 85.02 | 70.01 | 78.73 | 80.39 | 83.52 |
| 12 | 88.11 | 97.30 | 90.30 | 98.91 | 90.41 | 89.28 | 74.07 | 85.00 | 86.52 | 89.77 |
| 14 | 90.95 | 97.96 | 92.43 | 99.23 | 93.02 | 92.63 | 77.72 | 87.84 | 89.43 | 92.81 |
| 16 | 93.97 | 99.13 | 94.17 | 99.81 | 94.81 | 94.59 | 80.89 | 90.86 | 92.47 | 95.70 |
| 18 | 96.17 | 99.76 | 96.67 | 99.62 | 96.86 | 95.75 | 83.25 | 93.06 | 94.69 | 97.63 |
| 20 | 98.21 | 99.95 | 98.70 | 99.53 | 98.39 | 96.30 | 85.63 | 95.10 | 96.95 | 99.03 |

It could be seen that when the trend slope of the dissolution curve within 1-10 hours was between 5-5.6, the drug in the sustained-release layer in the tablet maintained zero-order release during the whole release process, and at least 95% of the drug was released at 20 hours. However, when the slope of dissolution curve was too small (less than 5.0), the release rate of the drug in the sustained-release portion was too slow, which would lead to incomplete dissolution of the drug within 20 hours and affect bioavailability; when the slope of the dissolution curve was too large (greater than 5.6), the drug in the sustained release portion would be released at a faster rate in the early stage, and the drug could not maintain nearly zero-order release in the later stage, which affected the sustained release effect.

The tablet 2-1 was released to 97% at 12 hours, because the API SR/IR was less than 2, and the API of the immediate-release layer accounted for 33% (completely released within 30-45 minutes), so the total API was released to 46% in the 1st hour, so that it was almost completely released by the 12th hour of the post-sustained release stage, but it was nearly zero-order release in the whole process within 1-12 hours.

## Claims

1. A biphasic controlled-release preparation, which consists of a drug-containing immediate-release layer (IR), a drug-containing sustained-release layer (SR) and a drug-free blocking layer (BL), wherein the drug-containing sustained-release layer (SR) is wrapped between the drug-containing immediate-release layer (IR) and the blocking layer (BL); and the drug-containing immediate-release layer (IR) is formed by compressing in a tableting manner and covering the drug-containing sustained-release layer (SR).

2. The biphasic controlled-release preparation according to claim 1, wherein the drug-containing immediate-release layer (IR) consists of the following components: an active substance, a filler, an adhesive, a disintegrant, a lubricant and a flow aid.

3. The biphasic controlled-release preparation according to claim 1, wherein the drug-containing sustained-release layer (SR) consists of the following components: an active substance, a sustained-release polymer, a filler, an adhesive, a lubricant and a flow aid; and the drug-containing sustained-release layer (SR) and the drug-free blocking layer (BL) constitute a partial coating structure with one side opening, wherein the drug-containing sustained-release layer (SR) serves as a tablet core, and the drug-free blocking layer (BL) functions as a partial coating layer.

4. The biphasic controlled-release preparation according to claim 1, wherein the drug-free blocking layer (BL) consists of the following components: a sustained-release polymer, a filler, an adhesive, a lubricant and a flow aid.

5. The biphasic controlled-release preparation according to any one of claims 1-4, wherein
a ratio of the mass of the drug-containing immediate-release layer (IR) to the mass of the drug-containing sustained-release layer (SR) is 5:1-1.5:1;
a ratio of the mass of the drug-containing immediate-release layer (IR) to the mass of the drug-free blocking layer (BL) is 1:1-0.125:1; and
a ratio of the mass of the drug-containing sustained-release layer (SR) to the mass of the drug-free blocking layer (BL) is 0.2:1-0.05:1.

6. The biphasic controlled-release preparation according to claim 5, wherein the content of a filler in the drug-containing sustained-release layer (SR) is 30-70%, and preferably 50%; and the material of the filler is selected from one or more of lactose, sucrose, calcium sulfate, calcium carbonate and calcium phosphate, and preferably lactose.

7. The biphasic controlled-release preparation according to claim 5, wherein the content of an adhesive in the drug-containing immediate-release layer (IR) is 3-10%, the content of an adhesive in the drug-free blocking layer (BL) is 2-10%, and the adhesive is selected from one or more of HPC, HPMC, povidone and sodium carboxymethyl cellulose; wherein the viscosity of HPC is 75-700 cps, and the viscosity of HPMC is 3-50 cps.

8. The biphasic controlled-release preparation according to claim 7, wherein the content of the adhesive in the drug-containing immediate-release layer (IR) is 5%, and the material is composed of HPMC and HPC in a content ratio of 0-1:1.

9. The biphasic controlled-release preparation according to claim 1, wherein the mass of an active substance in the drug-containing sustained-release layer is greater than that in the drug-containing immediate-release layer, and a ratio of the mass of the active substance in the drug-containing sustained-release layer to the mass of the active substance in the drug-containing immediate-release layer is ≤ 4; and preferably 4:1-1:1.

10. The biphasic controlled-release preparation according to any one of claims 3-9, wherein a disintegrant in the drug-containing immediate-release layer is selected from one or more of crosslinked sodium carboxymethyl starch, crosslinked sodium carboxymethyl cellulose, crospovidone PVPP and low-substituted hydroxypropyl cellulose L-HPC; and preferably crosslinked sodium carboxymethyl starch or crosslinked sodium carboxymethyl cellulose;
the lubricants in the drug-containing immediate-release layer (IR), the drug-containing sustained-release layer (SR) and the drug-free blocking layer (BL) are independently selected from one or more of magnesium stearate, stearic acid, sodium stearyl fumarate, glyceryl behenate, hydrogenated castor oil and sodium dodecyl sulfate; and preferably magnesium stearate or stearic acid;
the flow aids in the drug-containing immediate-release layer (IR), the drug-containing sustained-release layer (SR) and the drug-free blocking layer (BL) are independently colloidal silicon dioxide or talcum powder; and preferably colloidal silicon dioxide; and
the sustained-release polymer (skeleton material) in the drug-containing sustained-release layer (SR) and the drug-free blocking layer (BL) is hydroxypropyl methylcellulose.

11. The biphasic controlled-release preparation according to claim 10, wherein the content of the hydroxypropyl methylcellulose in the drug-containing sustained-release layer (SR) is 20.00-60.00%, and preferably 25.00-50.00%; and
the content of the hydroxypropyl methylcellulose in the drug-free blocking layer (BL) is 20.00-60.00%, and preferably 25.00-50.00%.

12. The biphasic controlled-release preparation according to claim 10, wherein the hydroxypropyl methylcellulose in the sustained-release polymer is selected from one or more of E30LV, E50LV, K100LV, K4M, K15M, K100M and xanthan gum.

13. The biphasic controlled-release preparation according to any one of claims 1-12, wherein in an *in vitro* dissolution test, at least 85% of active ingredients in the drug-containing immediate-release layer (IR) are dissolved within 45 min, and a trend slope of a dissolution curve of active ingredients in the drug-containing sustained-release layer (SR) within 1-10 hours is between 5.0-5.6, with at least 95% being released within 20 hours.

14. A method for preparing the biphasic controlled-release preparation according to any one of claims 1 to 13, comprising the following steps:
a. preparing granules of an immediate-release layer according to a formulation for later use;
b. preparing granules of a sustained-release layer according to a formulation for later use;
c. preparing granules of a blocking layer according to a formulation for later use;
d. placing the granules of the sustained-release layer into a punching die of a tablet press, and compressing the granules into a plain tablet core for later use; and
e. placing a formulation amount of the granules of the blocking layer into a punching die of a tablet press, placing a plain tablet core layer in the center of the granules of the blocking layer, and precompressing at a pre-compression pressure of 0.1-2 KN, so that the plain tablet core forms the drug-containing sustained-release layer (SR), and the plain tablet core is trapped in the granules of the blocking layer, and all faces except the top face of the plain tablet core are surrounded by the blocking layer, thereby forming a partial coating structure with one side opening; and then placing a formulation amount of the granules of the immediate-release layer into the punching die, and applying a main pressure of 5-35 KN, so that the granules of the immediate-release layer cover and adhere on the opening of the partial coating structure in a compression manner to form the drug-containing immediate-release layer (IR), thereby obtaining a finished tablet.

15. The method according to claim 14, wherein the steps a, b and c are specifically as follows:
a. weighing the active substance, the filler, the disintegrant and the adhesive material other than HPC in a formulation amount required for preparing the drug-containing immediate-release layer, carrying out wet granulation by addition of purified water or an alcohol solution, sieving wet granules through a 1,000-8,000 µm sieve, drying the wet granules, sieving dry wet granules through a 1,000-8,000 µm sieve, adding a flow aid, premixing, and then adding a lubricant and mixing to prepare granules of the immediate-release layer for later use;
b. weighing the active substance, the sustained-release polymer and the filler in a formulation amount required for preparing the drug-containing sustained-release layer, carrying out wet granulation with purified water or an alcohol solution, sieving wet granules through a 1,000-8,000 µm sieve, drying the wet granules, sieving dry granules through a 1,000-8,000 µm sieve, adding a flow aid, premixing, then adding a lubricant and mixing to prepare granules of the sustained-release layer for later use; and
c. weighing the sustained-release polymer, the filler and the adhesive other than HPC in a formulation amount required for preparing the blocking layer, fully mixing them, carrying out wet granulation by addition of purified water or an alcohol solution, sieving wet granules through a 1,000-8,000 µm sieve, drying the wet granules, sieving dry granules through a 1,000-8,000 µm sieve, adding a flow aid, premixing, then adding a lubricant and mixing to prepare granules of the blocking layer for later use; wherein in the step e, the pre-compression pressure is 0.1-0.5 KN; and a main pressure is 10-25 KN.

16. The method according to claim 15, wherein when the adhesive materials in the drug-containing immediate-release layer (IR) and the blocking layer comprise HPC, the HPC is added by being dissolved in the purified water or alcohol solution used for the wet granulation.
